# EUROPEAN PATENT SPECIFICATION

(11) **EP 1 946 119 B1**
(45) Date of publication and mention of the grant of the patent: **27.10.2010**
(21) Application number: 05808964.0
(22) Date of filing: 14.09.2005
(51) Int. Cl.: G01N 33/68

(54) **AN ADDITIVE SCORING METHOD FOR MODIFIED POLYPEPTIDE**
ADDITIVES WERTUNGSVERFAHREN FÜR MODIFIZIERTES POLYPEPTID
PROCEDE DE NOTATION ADDITIVE POUR POLYPEPTIDE MODIFIE

(30) Priority: 08.08.2005 KR 20050072198
(43) Date of publication of application: 23.07.2008
(73) Proprietor: Korea Basic Science Institute, Daejeon 305-333 (KR)
(72) Inventor: AHN, Yeong Hee, Chungcheongbuk-do 360-818 (KR); KWON, Kyung-Hoon, Daejeon-shi 302-753 (KR); YOO, Jong Shin, Daejeon-shi 305-308 (KR)
(74) Representative: Brady, Paul Andrew
(86) International application number: PCT/KR2005/003037
(87) International publication number: WO 2007/018327

(56) References cited:
- WO-A-02/31509
- WO-A-2004/061407
- US-A- 6 017 693
- MOORE R.E. ET AL.: 'Qscore: an algorithm for evaluating SEQUEST database search results' AMERICAN SOCIETY FOR MASS SPECTROMETRY vol. 13, 2002, pages 378 - 386, XP004346622
- YATES J.R. ET AL.: 'Method to correlate tandem mass spectra of modified peptides to amino acid sequences in the protein database' ANAL. CHEM. vol. 67, 1995, pages 1426 - 1436, XP002209924
- PEVZNER P.A. ET AL.: 'Efficiency of database search for identification of mutated and modified proteins via mass spectrometry' GENOME RESEARCH vol. 11, 2001, pages 290 - 299, XP002273859
- LIEBLER D.C. ET AL.: 'Peptide Sequence Motif Analysis of Tandem MS Data with the SALSA Algorithm' ANAL. CHEM. vol. 74, 2002, pages 203 - 210, XP003008579
- HAVILIO M. ET AL.: 'INTENSITY-BASED STATISTICAL SCORER FOR TANDEM MASS SPECTROMETRY' ANAL. CHEM. vol. 75, 2003, pages 435 - 444, XP001170893
- KAPP E.A. ET AL.: 'MINING A TANDEM MASS SPECTROMETRY DATABASE TO DETERMINE THE TRENDS AND GLOBAL FACTORS INFLUENCING PEPTIDE FRAGMENTATION' ANAL. CHEM. vol. 75, 2003, pages 6251 - 6264, XP009058388

## Description

### Technical Field

The present invention relates to a method for identifying modified polypeptide sequence and modified information thereof. More precisely, the invention relates to a method for identifying modified peptide in which fragment ion signals are obtained from target peptide precursor by using tandem mass spectrometry, candidate peptides and every possible peptide fragmentation patterns thereof are designed based on the mass data thereby, match scores corresponding to each fragmentation pattern of candidate peptides are given, and then by combining each matching scores derived from possible fragmentation patterns of a candidate peptide, modified target polypeptide is identified by tandem mass data based on the provided match score.

### Background Art

Mass spectrometry is one of the key methods analyzing biopolymer, and has been taking a major part in proteomics study together with protein database searching. In particular, tandem mass spectrometry is very effective for the direct identification of protein or peptide sequences (McCormack et al., Anal. Chem., 69: 767-776, 1997). Tandem mass spectrometry data include the information about mass of parent peptide and masses of each fragment of the peptide at the same time. Therefore, expected sequence of the sample peptide can be obtained by comparing peptide fragment spectrum with theoretical spectra generated from protein sequences of the protein database. Then, the matching degree between experimental spectrum and possible theoretical spectrum is scored. Among peptides showing match scores, the one showed the highest match score is selected to identify the sequence of a sample peptide.

In tandem mass spectrometry, the scoring system has to assign higher scores to the better matched sequences. Among the possible sequences predicted from large database, the highest score match must be the truly assigned sequence. The scoring algorithms pursue to minimize false positive selections. MS/MS spectrum of peptides generated from low abundant proteins in a sample has low quality in general, resulting in low match scores. It is hard to identify such peptides, compared with those generated from major proteins of a sample. In order to overcome this problem, various scoring methods were proposed (Bafna et al., Bioinformatics, 17: S13-S21, 2001; Dancik et al., J. Comp. Biol., 6: 327-342, 1999; Havilio et al., Anal. Chem., 75: 435-444, 2003; Li et al., Rapid Commun. Mass Spectrom., 18: 1655-9, 2004). And various search systems have been used such as Mascot (Pappin et al., Curr. Biol., 3: 327-332, 1993), Sequest (Eng et al., J. Am. Soc. Mass Spectrom., 5: 976-989, 1994 and US Patent no. 6,017,693), Sonar (Field et al., Proteomics, 2: 36-47, 2002) and X! Tandem (Craig et al., Bioinformatics, 20: 1466-7, 2004).

The mentioned systems have been developed considering possible modifications of peptides, for example, the modifications by methylation, acetylation, cysteine alkylation, oxidation, phosphorylation or chemical labeling, etc. However, since a variety of factors, besides the above mentioned, affect peptide fragment spectrum, it is required to develop an improved method that can minimize false positive selection (Kapp et al., Anal. Chem., 75: 6251-6264, 2003). New systems enabling automatic validation of results obtained from several searching system have been developed (Elias et al., Nat. Biotechnol., 22: 214-219, 2004; Anderson et al., J. Proteome Res., 2: 137-146, 2003; Keller et al., Anal. Chem., 74: 5385-5392, 2002; Sadygov et al., J. Proteome Res., 1: 211-215, 2002; Moore et al., J. Am. Soc. Mass Spectrom., 13: 378-386, 2002), but they still have some limitations on the confident identification of modified peptides, in particular, chemically modified peptides showing various fragmentation patterns in MS/MS analysis.

To identify the peptide sequences including modified or tagged sites thereof from tandem mass data with high reliability, the present inventors considered every possible peptide fragmentation patterns using fragment ion signals obtained by tandem mass spectrometry from target peptide precursors. They gave match scores to each candidate peptide based on the above obtained patterns, and then integrated the produced scores, resulting in the development of a method for identifying modified peptides having different fragmentation patterns. The present inventors finally completed this invention by confirming that the method of the invention can be more effectively used with excellent searching efficiency and high reliability for the identification of modified peptides since it counts the meaningful spectrum peaks that were missed by the other algorithms by integrating various different fragmentation patterns of a candidate peptide.

### Disclosure of Invention

### Technical Problem

It is an object of the present invention to provide a method for identifying polypeptide sequences and modified information thereof using mass analysis data.

### Technical Solution

The present invention provides a method for analyzing modified polypeptide sequence and modified information thereof using an additive scoring method of match scores obtained from mass analysis data, comprising the following steps:
1) Making a list of promising candidate peptides by comparing the degree of match between experimental peptide mass obtained from polypeptide precursors and theoretical peptide mass produced from protein sequence database;
2) Scoring the degree of match by comparing a list of experimental peptide fragment masses and a list of theoretical peptide fragment masses prepared from the promising candidate peptide fragment patterns of the above step 1);
3) Selecting the candidate peptide showing the highest match score;
4) Additionally scoring the degree of match between experimental polypeptide fragment masses and theoretical fragment masses generated by fragment patterns different from the patterns of the candidate peptide selected in the above step 3);
5) Adding up the highest match score obtained in the step 3) and the additional match scores prepared in the step 4); and,
6) Reporting the candidate peptide selected from the added scores of the step 5).

The present invention also provides a method for analyzing modified polypeptide sequence and modified information thereof comprising the following steps:
1) Making a list of promising candidate peptides by comparing the degree of match between experimental peptide mass obtained from polypeptide precursors and theoretical peptide mass produced from protein sequence database;
2) Scoring the degree of match by comparing a list of experimental peptide fragment masses and a list of theoretical peptide fragment masses prepared from the promising candidate peptide fragment patterns of the above step 1);
3) Adding up the match scores of different fragmentation patterns of each candidate peptides;
4) Selecting the candidate peptide showing the highest match score; and,
5) Reporting the candidate peptide selected as the polypeptide of the invention.

Hereinafter, the present invention is described in detail. ,

The present invention provides a method for screening modified peptides that may show a variety of peptide fragmentation patterns during the process for the tandem mass spectrometry. In the invention, every possible peptide fragmentation pattern is scanned from fragment ion signals, and the matched peaks for each pattern are involved in scoring, and its score is added to the peptide match score.

The present invention developed a new method for analyzing modified polypeptide sequence and modified information thereof with enhanced efficiency and reliability using mass data.

Particularly, the method of the present invention for analyzing modified polypeptide is composed of the following steps:

1) Making a list of promising candidate peptides by comparing experimental peptide mass obtained from polypeptide precursor with the theoretical peptide masses produced from protein sequence database;

2) Scoring the degree of match by comparing the list of experimental peptide fragment masses and the other list of theoretical peptide fragment masses prepared from each fragmentation pattern of the promising candidate peptides of the above step 1);

3) Selecting the candidate peptide showing the highest match score;

4) Scoring the degree of match between experimental polypeptide fragment masses and theoretical fragment masses generated by fragment patterns different from the patterns of the candidate peptide selected in the above step 3);

5) Adding up the highest match score obtained in the step 3) and the additive match score prepared in the step 4); and

6) Reporting final candidate peptides selected from the added scores of the step 5).

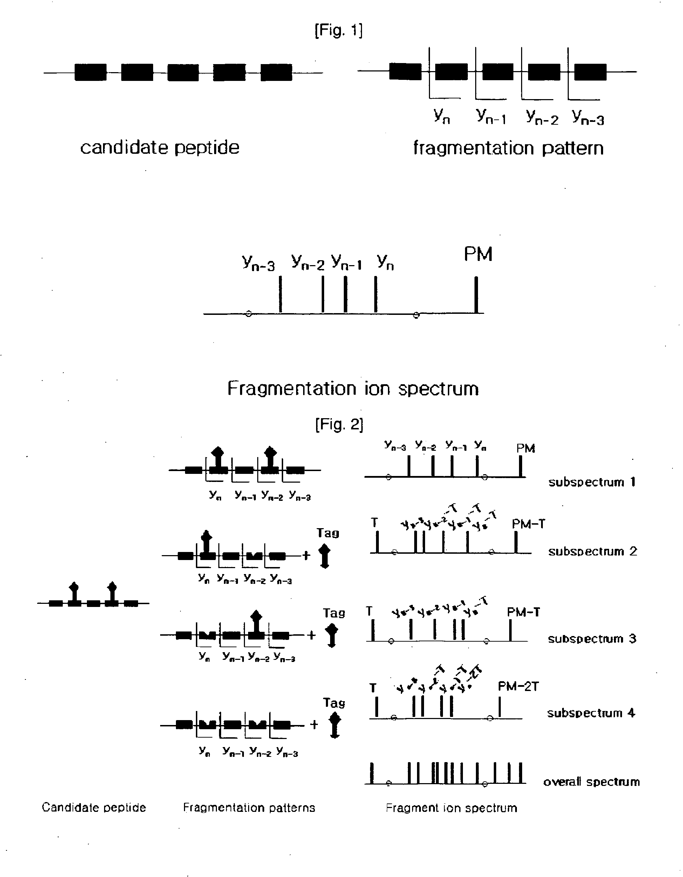

In general, mass spectrometer is an important tool for the analysis of biopolymer, particularly protein. By mass spectrometry, molecular weight of a protein or peptide generated from the protein is measured by the ratio of mass to charge (m/z), and in particular by tandem mass spectrometry, internal sequence of a biopolymer can be analyzed. In tandem mass spectrometry, selected target peptide precursors or protein precursors are split into various fragment patterns in gas phase, resulting in MS/MS spectrum comprising complicated fragmentation signals (see Fig. 1). The produced peptide fragment spectra are compared with the spectra theoretically generated from protein sequences stored in protein database to select the best match pattern among many peptide fragment patterns, by which sample peptide sequence is identified. At this time, the degree of match between experimental spectrum and theoretical spectrum was measured and expressed by score values.

Modification in chemical structure of peptide or protein, especially amino acid residues, accompanies not only the cut of backbone of peptide but also the cut of side chains of amino acid residues during the fragmentation of peptide in gas phase (see Fig 2). The modification in chemical structure arises from the post-translational modification by the biological process or some artificial chemical or biological treatment by analytical experiment. Modifications occurring in side chains of amino acid residues during the fragmentation in gas phase, are exemplified by deamination, demethylation, deglycosylation, or dephosphorylation. In addition, various fragmentation petterns, for example, de-tagging when a peptide is labeled with a tag, might be observed together with backbone cut.

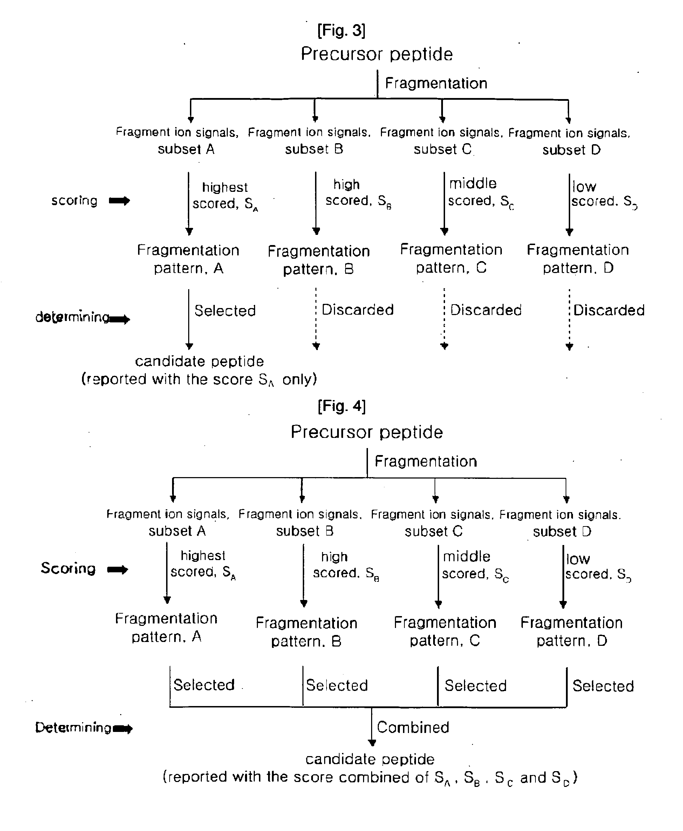

The resultant MS/MS spectrum shows complicated fragmentation signals that include the detailed information on the peptide structure. Even a peptide having the same peptide sequence can produce different fragmentation signals according to fragmentation patterns. The different fragmentation signals are divided into subsets according to the fragment pattern, which is used for the identification of peptide sequence. Nevertheless, generally used screening method for peptide sequence is that experimental spectrum is compared with theoretical spectrum generated from protein sequence listed in protein database and then a pattern showing the highest match from the comparison is selected among possible peptide fragment patterns to identify peptide sequence of a sample (see Fig. 3). But, this conventional method has a disadvantage of neglecting the information possessed by different fragmentation signals produced by minor fragment patterns. Those excluded fragmentation signals might have information on peptide structure, in particular information on modification region. Thus, a new scoring method is required where the fragmentation signals by the minor fragmentation patterns are participating at scoring to concern more detailed information on the peptide structure.

The present inventors developed a new method which characteristically detects all different fragmentation signals having the same sequence as that of the peptide showing the highest match score but generated by different fragmentation patterns (see Fig. 4).

Particularly, to identify the sequence of a sample peptide, experimental spectrum is compared with theoretical spectrum generated from protein sequences stored at protein database, and then fragmentation pattern A showing the highest match score is selected among every possible peptide fragmentation patterns. Then, fragmentation signals having the same sequence as that of identified peptide but generated by different fragmentation patterns B, C, and D are divided into subsets of B, C, D, followed by numerical evaluation with each subset spectra. Among fragmentation signals of each subset, those selected first from fragment pattern A were either excluded from numerical evaluation or weight was lowered. To calculate match score, peptide cut styles (for example, a, b, c, x, y, z, etc) can have priorities and in that case, repeated scoring to a specific signal is avoided or weight to the signal is lowered. The specific signals characteristically generated by fragmentation of side chains of amino acid residues forming such signals as T, PM-T, and PM-2T as shown in Fig. 2 can get special weight. The above characteristic signals can be used as a tag for analyzing modified peptides. Match scores between patterns are added up to calculate match scores of peptide. While match scores obtained from each fragmentation patterns are added up, proper weight can be given to each step of detecting match of each fragmentation pattern.

The method for screening peptides and sequence analysis of the present invention enables the analysis of those fragmentation signals generated even minor fragmentation patterns, enhancing the screening efficiency of peptide, particularly efficiency in analysis of modified peptides having different fragmentation patterns.

The other advantage of the method of the invention is usefulness in screening peptides specifically generated by site-specific proteolysis developed for the analysis of modified protein body. Site-specific proteolysis is the way to substitute modified site of a target protein or a peptide, particularly phosphorylation or glycosylation sites, into protease-recognizable site, and then induce enzymatic hydrolysis therein to afford peptide having the modified site with C-terminal residue. For example, modified serine or threonine site is substituted with aminoethanethiol tag (Knight et al., Nat. Biotechnol., 21: 1047-1396, 2003) or guanidinoethanethiol tag (Ahn et al., PCT/ KR2004/000256). However, data from tandem mass spectrometry prepared by the mentioned site-specific proteolysis include the information on peptides generated by hydrolysis at tagged site, indicating that the data of peptides in which tagged amino acid residues are located at C-terminus. Although such specific peptides provide very useful information on localization of protein modification, any screening program has not been totally successful for interpretation of the information, so far. Therefore, the present inventors developed a screening program that can screen not only well known K-terminal or R-terminal peptides but also peptides containing tagged residue at the C-terminus. The present inventors further increased the efficiency of the search method for modified protein and peptide, especially modified site-terminated peptide, by the additive scoring method mentioned above.

### Brief Description of the Drawings

Fig. 1 is a schematic diagram showing the fragmentation patterns cut from precursor peptide sequence by tandem mass spectrometry, and the resultant hypothetical MS/MS spectrum,

PM: Signal caused by precursor mass,

Fig. 2 is a schematic diagram showing that not only sequence backbone of precursor peptide but also side chains of amino acids forming a peptide can be cut into small fragments through tandem mass spectrometry, and the resultant hypothetical MS/MS spectrum,

T: Signal caused by a part of side chain of amino acid or a fragment of tag linked to a peptide,

Fig. 3 is a schematic diagram showing the conventional non-additive peptide screening process,

Fig. 4 is a schematic diagram showing the peptide screening by the additive scoring method of the present invention,

Fig. 5 is an example searched by the conventional non-additive scoring method for the result of tandem mass spectrometry of casein alpha-S1 tryptic peptide (sequence 58-73, DIGS*ES*TEDQAMEDIK, m/z 1969.8737),

Fig. 6 is an example searched by the newly invented additive scoring method for the result of tandem mass spectrometry of casein aloha-S1 tryptic peptide (sequence 58-73, DIGS*ES*TEDQAMEDIK, m/z 1969.8737),

Fig. 7 shows that the result of tandem mass spectrometry with casein alpha-S1 tryptic peptides was screened by the conventional non-additive scoring method,

S*: Guanidinoethylcysteine site originated from phosphoserine,

Fig. 8 shows that the result of tandem mass spectrometry with casein alpha-S1 tryptic peptides was screened by the additive scoring method of the present invention,

V: Peptide screened with an increased search score by the additive scoring method,

Fig. 9 is an example searched by the conventional non-additive scoring method for the result of tandem mass spectrometry of beta-casein tryptic peptide (sequence 33-48, FQS*EEQQQTEDELQDK, m/z 2082.8972),

Fig. 10 is an example searched by the invented additive scoring method for the result of tandem mass spectrometry of beta-casein tryptic peptide (sequence 33-48, FQS*EEQQQTEDELQDK, m/z 2082.8972).

### Best Mode for Carrying Out the Invention

Practical and presently preferred embodiments of the present invention are illustrative as shown in the following Examples.

### <Example 1> Analysis of modified polypeptide sequence and modified information using mass analysis data

Following analysis was performed using tandem mass data to evaluate the additive scoring method. First, tandem mass spectrometry data was obtained by guanidinoethanethiol tagging/digestion method, which is described in PCT/KR2004/000256. The document is used as a reference for this invention. Guanidinoethanethiol tagging/ digestion method is useful for high-sensitive analysis for modified peptides, and particularly guanidinoethanethiol (GET) labeled peptides generated by the method show various fragmentation patterns through tandem mass spectrometry.

The present inventors compared the additive scoring method of the present invention with the conventional non-additive scoring method. Scoring is done with a differential weight according to fragment ion types measured in mass analysis and mass analysis systems used. That is, in the case of fragments obtained from CID (collision induced dissociation), a peak matching with y or b ion series received higher match score. In the meantime, in the case of fragments obtained from ECD (electron capture dissociation), a peak matching with c or z ion series received higher match score.

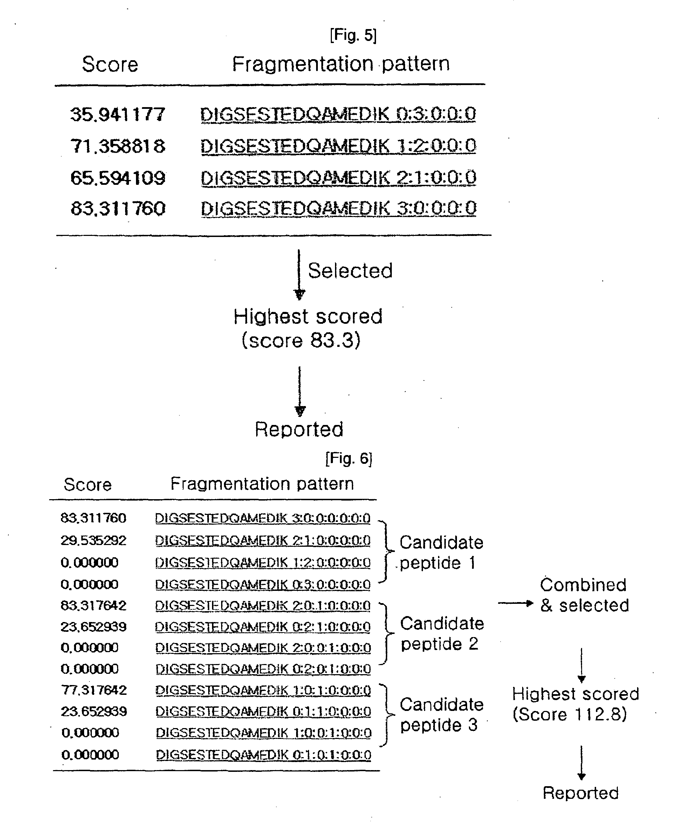

Fig. 5 is an example searched by the conventional non-additive scoring method for the result of tandem mass spectrometry of casein alpha-S1 tryptic peptide (sequence 58-73, DIGS*ES*TEDQAMEDIK, m/z 1969.8737). S* was originally phosphoserine site, but was changed into guanidinoethylcysteine site by the guanidinoethanethiol tagging/digestion method. The peptide contains two labeled sites which are breakable into fragments by tandem mass spectrometry. As shown in Fig. 5, the conventional peptide screening method can select the one that shows the highest match score (83.3) to report. At this time, lower match scores which are scored by the peptide having the same amino acid sequence but different fragmentation patterns are discarded. As a result, some useful fragmentation signals in the mass data regarding protein structure can be discarded. The method of the present invention, an additive scoring method, overcame the problem. Fig. 6 is an example searched by the invented additive scoring method for the result of tandem mass spectrometry of casein alpha-S 1 tryptic peptide (sequence 58-73, DIGS*ES*TEDQAMEDIK, m/z 1969.8737). Three potential candidate peptides were recorded considering the locations theoretically possible for GET-labeling (Ser61, Ser63 and Thr64). Match scores were obtained from potential candidate peptides according to every possible fragmentation patterns. Then, lower match score (29.5) was added up, rather than higher match score (83.3), resulting in the combined score (112.8) as the match score of peptide. At this time, repeated scoring for a fragment ion mass should be avoided.

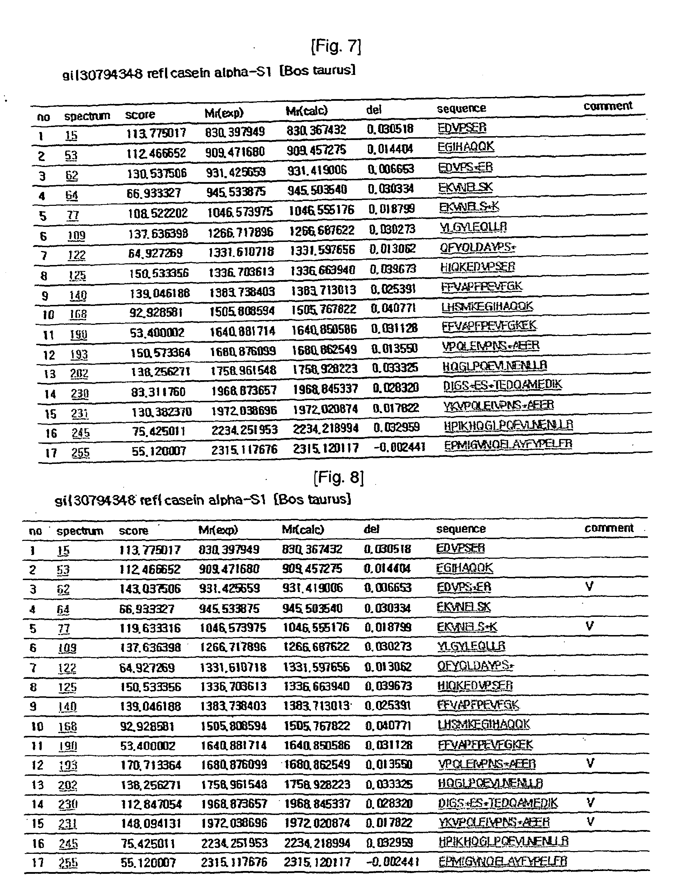

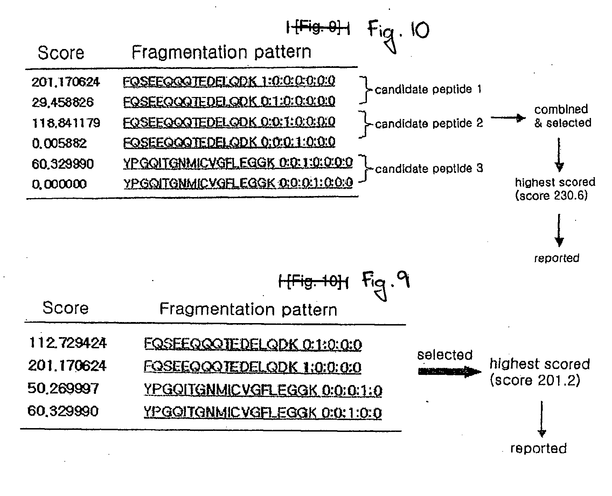

Fig. 7 and Fig. 8 show the search results by both the conventional scoring method and the additive scoring method of the present invention respectively for mass data obtained from tandem mass analysis of tryptic peptides of GET-labeled casein alfa-S1. Peptides, corresponding to peptides (V) no. 3, 5, 12, 14 and 15 of Fig. 8, showed increased search scores by the additive scoring method of the invention, and they were all GET-labeled peptides phosphorylated formerly. In the meantime, the scores of those peptides who did not contain modified residue remained unchanged. The above results indicate that the method of the present invention is very efficient in screening modified peptides. Fig. 9 and Fig. 10 show the search results by both the conventional scoring method and the additive scoring method of the present invention respectively for mass data obtained from tandem mass analysis of tryptic peptide (sequence 33-48, FQS*EEQQQTEDELQDK, m/z 2082.8972) of GET-labeled beta-casein. The peptide was searched with the match score, 230.6 (Fig. 10) by the additive scoring method, but with the match score, 210.2 (Fig. 9) by the non-additive scoring method. It also confirms that the method of the present invention produces better search result.

### Industrial Applicability

[74] As explained hereinbefore, the present invention provides an efficient method for analyzing complex mass data obtained from tandem mass analysis of modified polypeptides which produce a variety of fragment ions by peptide backbone cleavage and/or side-chain cleavage on modified residue during mass analysis. Thus, searching efficiency and reliability of the resulting search result for tandem mass data of modified peptides can be enhanced by the method of the present invention.

## Claims

1. A method for analyzing modified polypeptide sequence and modified information thereof using an additive scoring method of match scores obtained from mass analysis data, comprising the following steps:
1) Making a list of promising candidate peptides by comparing the degree of match between experimental peptide mass obtained from polypeptide precursors and theoretical peptide mass produced from protein sequence database;
2) Scoring the degree of match by comparing a list of experimental peptide fragment masses and a list of theoretical peptide fragment masses prepared from the promising candidate peptide fragment patterns of the above step 1);
3) Selecting the candidate peptide showing the highest match score;
4) Additionally scoring the degree of match between experimental polypeptide fragment masses and theoretical fragment masses generated by fragment patterns different from the patterns of the candidate peptide selected in the above step 3);
5) Adding up the highest match score obtained in the step 3) and the additional match scores prepared in the step 4); and,
6) Reporting the candidate peptide selected from the added scores of the step 5).

2. The method as set forth in claim 1, wherein the polypeptide is obtained by chemical or enzymatic hydrolysis of protein.

3. The method as set forth in claim 2, wherein the protein is modified by post-translational modification in biological media or chemical reaction during analyzing process.

4. The method as set forth in any of claims 1 to 3, wherein the polypeptide is peptide labeled with a tag selected from a group consisting of guanidinoethanethiol, aminoethanethiol, mercaptoethanol, alkoxyethanethiol, alkane thiol and biotin linked alkane thiol.

5. The method as set forth in any of claims 1 to 4, wherein the masses of experimental peptide fragments are obtained from fragment ions generated simultaneously by tandem mass spectrometry.

6. The method as set forth in any of claims 1 to 5, wherein the experimental fragment masses matched and scored in steps 2) and 3) is not counted again in step 4).

7. A method for analyzing modified polypeptide sequence and modified information thereof comprising the following steps:
1) Making a list of promising candidate peptides by comparing the degree of match between experimental peptide mass obtained from polypeptide precursors and theoretical peptide mass produced from protein sequence database;
2) Scoring the degree of match by comparing a list of experimental peptide fragment masses and a list of theoretical peptide fragment masses prepared from the promising candidate peptide fragment patterns of the above step 1);
3) Adding up the match scores of different fragmentation patterns of each candidate peptides;
4) Selecting the candidate peptide showing the highest match score; and,
5) Reporting the candidate peptide selected as the polypeptide of the invention.

8. The method as set forth in claim 7, wherein the polypeptide is obtained by chemical or enzymatic hydrolysis of protein.

9. The method as set forth in claim 8, wherein the protein is modified by post-translational modification in biological media or chemical reaction during analyzing process.

10. The method as set forth in any of claims 7 to 9, wherein the polypeptide is peptide labeled with a tag selected from a group consisting of guanidinoethanethiol, aminoethanethiol, mercaptoethanol, alkoxyethanethiol, alkane thiol and biotin linked alkane thiol.

11. The method as set forth in any of claims 7 to 10, wherein the masses of experimental peptide fragments are obtained from fragment ions generated simultaneously by tandem mass spectrometry.

12. The method as set forth in any of claims 7 to 11, wherein in step 3), the repeated each match between experimental peptide fragments and theoretical peptide fragments generated from fragmentation patterns of a candidate peptide is scored once only.

## Patentansprüche

1. Verfahren zum Analysieren von einer modifizierten Polypeptid-Sequenz und einer modifizierten Information daraus unter Verwendung eines additiven Wertungsverfahrens von Kompatibilitäts-Punkten, welche aus Massenanalysedaten erlangt sind, welches die folgenden Schritte enthält:
1) Erstellen von einer Liste von erfolgversprechenden Kandidaten-Peptiden durch ein Vergleichen des Kompatibilitätsgrades zwischen einer experimentellen Peptid-Masse, welche aus Polypeptid-Vorläufern erlangt ist, und einer theoretischen Polypeptid-Masse, welche aus einer Protein-Sequenz-Datenbasis erzeugt ist;
2) Bewerten des Kompatibilitätsgrades durch Vergleichen einer Liste von experimentellen Peptid-Fragment-Massen und einer Liste von theoretischen Peptid-Fragment-Massen, welche aus den erfolgversprechenden Kandidat-Peptid-Fragment-Mustern des obigen Schrittes 1) vorbereitet sind;
3) Auswählen des Kandidat-Peptids, welches den höchsten Kompatibilitäts-Punkt zeigt;
4) zusätzliches Bewerten des Kompatibilitätsgrades zwischen experimentellen Polypeptid-Fragment-Massen und theoretischen Fragment-Massen, welche durch Fragment-Muster erzeugt sind, welche sich von den Mustern des Kandidat-Peptids unterscheiden, welches im obigen Schritt 3) ausgewählt ist;
5) Aufaddieren des höchsten Kompatibilitäts-Punktes, welcher in Schritt 3) erlangt ist, und der zusätzlichen Kompatibilitäts-Punkte, welche in Schritt 4) vorbereitet sind; und
6) Melden des Kandidaten-Peptids, welches aus den addierten Bewertungen des Schritts 5) ausgewählt ist.

2. Verfahren nach Anspruch 1, bei welchem das Polypeptid durch eine chemische oder enzymatische Hydrolyse eines Proteins erlangt wird.

3. Verfahren nach Anspruch 2, bei welchem das Protein durch eine Nachüberführungs-Modifikation in biologische Medien oder eine chemische Reaktion während eines Analyseprozesses modifiziert wird.

4. Verfahren nach einem der Ansprüche 1 bis 3, bei welchem das Polypeptid ein Peptid ist, welches mit einer Kennzeichnung **gekennzeichnet** ist, welche aus einer Gruppe ausgewählt ist, welche Guanidinoethanethiol, Aminoethanethiol, Mercaptoethanol, Alkoxyethanethiol, Alkanthiol und Biotin-verbundenes Alkanthiol enthält.

5. Verfahren nach einem der Ansprüche 1 bis 4, bei welchem die Massen von experimentellen Peptid-Fragmenten aus Fragment-Ionen erlangt sind, welche gleichzeitig durch eine Tandem-Massen-Spektrometrie erzeugt sind.

6. Verfahren nach einem der Ansprüche 1 bis 5, bei welchem die experimentellen Fragment-Massen, welche in Schritten 2) und 3) abgestimmt und bewertet sind, in Schritt 4) nicht abermals gezählt werden.

7. Verfahren zum Analysieren von einer modifizierten Polypeptid-Sequenz und einer modifizierten Information daraus, welches die folgenden Schritte enthält:
1) Erstellen von einer Liste von erfolgversprechenden Kandidaten-Peptiden durch ein Vergleichen des Kompatibilitätsgrades zwischen einer experimentellen Peptid-Masse, welche aus Polypeptid-Vorläufern erlangt ist, und einer theoretischen Polypeptid-Masse, welche aus einer Protein-Sequenz-Datenbasis erzeugt ist;
2) Bewerten des Kompatibilitätsgrades durch Vergleichen einer Liste von experimentellen Peptid-Fragment-Massen und einer Liste von theoretischen Peptid-Fragment-Massen, welche aus den erfolgversprechenden Kandidat-Peptid-Fragment-Mustern des obigen Schrittes 1) vorbereitet sind;
3) Aufaddieren der Kompatibilitäts-Punkte von unterschiedlichen Fragmentierungsmustern von jedem Kandidaten-Peptid;
4) Auswählen jenes Kandidaten-Peptids, welches den höchsten Kompatibilitäts-Punkt aufzeigt; und
5) Melden jenes Kandidaten-Peptids, welches als das Polypeptid der Erfindung ausgewählt ist.

8. Verfahren nach Anspruch 7, bei welchem das Polypeptid durch eine chemische oder enzymatische Hydrolyse eines Proteins erlangt wird.

9. Verfahren nach Anspruch 8, bei welchem das Protein durch eine Nachüberführungs-Modifikation in biologische Medien oder eine chemische Reaktion während eines Analyseprozesses modifiziert wird.

10. Verfahren nach einem der Ansprüche 7 bis 9, bei welchem das Polypeptid ein Peptid ist, welches mit einer Kennzeichnung **gekennzeichnet** ist, welche aus einer Gruppe ausgewählt ist, welche Guanidinoethanethiol, Aminoethanethiol, Mercaptoethanol, Alkoxyethanethiol, Alkanthiol und Biotin-verbundenes Alkanthiol enthält.

11. Verfahren nach einem der Ansprüche 7 bis 10, bei welchem die Massen von experimentellen Peptid-Fragmenten aus Fragment-Ionen erlangt sind, welche gleichzeitig durch eine Tandem-Massen-Spektrometrie erzeugt sind.

12. Verfahren nach einem der Ansprüche 7 bis 11, bei welchem in Schritt 3) die wiederholte Bewertung zwischen experimentellen Peptid-Fragmenten und theoretischen Peptid-Fragmenten, welche aus Fragmentierungs-Mustern von einem Kandidaten-Peptid erzeugt sind, lediglich einmal bewertet wird.

## Revendications

1. Procédé pour analyser une séquence polypeptidique modifiée et des informations modifiées de celle-ci en utilisant un procédé d'attribution de scores additif de scores de correspondance obtenus à partir de données d'une analyse de masse, comprenant les étapes suivantes :
1) établir une liste de peptides candidats prometteurs en comparant le degré de correspondance entre une masse de peptides expérimentaux obtenue à partir de précurseurs polypeptidiques et une masse de peptides théoriques produite par une base de données de séquences protéiques ;
2) attribuer un score au degré de correspondance en comparant une liste de masses de fragments peptidiques expérimentaux et une liste de masses de fragments peptidiques théoriques préparée à partir des motifs des fragments peptidiques candidats prometteurs de l'étape 1) ci-dessus ;
3) sélectionner le peptide candidat montrant le score de correspondance le plus élevé ;
4) attribuer en outre un score au degré de correspondance entre les masses des fragments polypeptidiques expérimentaux et les masses des fragments théoriques générées par des motifs de fragments qui sont différents des motifs du peptide candidat sélectionné à l'étape 3) ci-dessus ;
5) additionner le score de correspondance le plus élevé obtenu à l'étape 3) et les scores de correspondance additionnels préparés à l'étape 4) ;
6) rapporter le peptide candidat choisi à partir des scores additionnés de l'étape 5).

2. Procédé selon la revendication 1, dans lequel le polypeptide est obtenu par une hydrolyse chimique ou enzymatique d'une protéine.

3. Procédé selon la revendication 2, dans lequel la protéine est modifiée par une modification post-traductionnelle dans un milieu biologique ou une réaction chimique pendant le processus d'analyse.

4. Procédé selon l'une quelconque des revendications 1 à 3, dans lequel le polypeptide est un peptide marqué avec un marqueur choisi dans un groupe consistant en le guanidino-éthanethiol, l'amino-éthanethiol, le mercaptoéthanol, l'alcoxy-éthanethiol, un alcane-thiol et un alcane-thiol lié à la biotine.

5. Procédé selon l'une quelconque des revendications 1 à 4, dans lequel les masses des fragments peptidiques expérimentaux sont obtenues à partir d'ions des fragments générés simultanément par une spectrométrie de masse en tandem.

6. Procédé selon l'une quelconque des revendications 1 à 5, dans lequel les masses des fragments expérimentaux présentant une correspondance et recevant un score aux étapes 2) et 3) ne sont pas de nouveau comptabilisées à l'étape 4).

7. Procédé pour analyser une séquence polypeptidique modifiée et des informations modifiées de celle-ci, comprenant les étapes suivantes :
1) établir une liste de peptides candidats prometteurs en comparant le degré de correspondance entre une masse de peptides expérimentaux obtenue à partir de précurseurs polypeptidiques et une masse de peptides théoriques produite par une base de données de séquences protéiques ;
2) attribuer un score au degré de correspondance en comparant une liste de masses de fragments peptidiques expérimentaux et une liste de masses de fragments peptidiques théoriques préparée à partir des motifs des fragments peptidiques candidats prometteurs de l'étape 1) ci-dessus ;
3) additionner les scores de correspondance des différents motifs de fragmentation de chaque peptide candidat ;
4) choisir le peptide candidat présentant le score de correspondance le plus élevé ; et,
5) rapporter le peptide candidat sélectionné en tant que polypeptide de l'invention.

8. Procédé selon la revendication 7, dans lequel le polypeptide est obtenu par une hydrolyse chimique ou enzymatique d'une protéine.

9. Procédé selon la revendication 8, dans lequel la protéine est modifiée par une modification post-traductionnelle dans un milieu biologique ou une réaction chimique pendant le processus d'analyse.

10. Procédé selon l'une quelconque des revendications 7 à 9, dans lequel le polypeptide est un peptide marqué avec un marqueur choisi dans un groupe consistant en le guanidino-éthanethiol, l'amino-éthanethiol, le mercaptoéthanol, l'alcoxy-éthanethiol, un alcane-thiol et un alcane-thiol lié à la biotine.

11. Procédé selon l'une quelconque des revendications 7 à 10, dans lequel les masses des fragments peptidiques expérimentaux sont obtenues à partir d'ions des fragments générés simultanément par une spectrométrie de masse en tandem.

12. Procédé selon l'une quelconque des revendications 7 à 11, dans lequel à l'étape 3), chaque correspondance répétée entre les fragments peptidiques expérimentaux et les fragments peptidiques théoriques générés à partir des motifs de fragmentation d'un peptide candidat reçoit un score une seule fois.
